# EUROPÄISCHE PATENTSCHRIFT

(11) **EP 1 256 838 B1**
(45) Veröffentlichungstag und Bekanntmachung des Hinweises auf die Patenterteilung: **06.04.2005**
(21) Anmeldenummer: 02405381.1
(22) Anmeldetag: 10.05.2002
(51) Int. Cl.: G03B 42/04

(54) **Filmhalter für die Dentaltechnik**
Film holder in dental technique
Monture de film dans la technique dentaire

(30) Priorität: 10.05.2001 CH 8482001
(43) Veröffentlichungstag der Anmeldung: 13.11.2002
(73) Patentinhaber: KerrHawe SA, 6934 Bioggio (CH)
(72) Erfinder: Da Rold, Marco, 6947 Vaglio (CH); Klauser, Rolf M., 6010 Kriens (CH); Kilcher, Beat, 6935 Bosco Luganese (CH); Keller, Wilhelm A., 6402 Merlischachen (CH)
(74) Vertreter: AMMANN PATENTANWAELTE AG BERN

(56) Entgegenhaltungen:
- GB-A- 1 043 495
- US-A- 4 592 084
- US-A- 5 022 065
- US-A- 5 737 388

## Beschreibung

Die vorliegende Erfindung bezieht sich auf einen Filmhalter für die Dentaltechnik gemäss Oberbegriff von Patentanspruch 1. Ein solcher Filmhalter ist in der EP-B-397 599 oder GB 1,043,495 beschrieben. Der dort beschriebene und seit langem im Handel sich befindliche Filmhalter ist als Röntgenfilmhalter ausgebildet und weist unter anderem eine Visiervorrichtung auf. Der Röntgenfilmhalter gemäss Stand der Technik weist eine U-förmige Filmklemme auf, die eine Rückenplatte sowie einen Klemmschenkel aufweist. Dabei entspricht die Rückenplatte in ihren Abmessungen genau dem Filmformat Nummer 0.

Die U-förmige Version der Filmklemme hat den Nachteil, dass die Klemmwirkung nur in einem einzigen Idealfall auf der ganzen Filmfläche zustande kommt, nämlich dann, wenn die Dicke des Filmes mit dem Krümmungswinkel der U-förmigen Verbindung der beiden Schenkel und im Abstand der beiden Schenkel voneinander in Übereinstimmung ist. In letzter Zeit werden die herkömmlichen, relativ dicken Röntgenfilme immer häufiger durch sog. Speicherfolien ersetzt. Diese bestehen aus einem sehr dünnen Trägermaterial, das mit einer strahlenempfindlichen Speicherschicht ausgestattet ist. Diese Speicherfolien sind nicht nur sehr dünn sondern weisen auch eine sehr glatte Oberfläche auf. Ausserdem darf die Oberfläche keinen mechanischen Reizen oder Verletzungen ausgesetzt werden, da solche zu falschen Signalen im Scanner und Computer führen und somit zu einem defekten Bild am Bildschirm und dann zu Falschinformationen.

Es ist von diesem Stand der Technik ausgehend Aufgabe der vorliegenden Erfindung, einen Filmhalter mit einer Filmklemme anzugeben, der nicht nur auf die verschiedenen Dicken der Filmen und von Speicherfolien sondern auch auf eventuell vorliegenden Dickenunterschiede und Aussenwülste sowie auf die besonderen Eigenschaften von Speicherfolien Rücksicht nimmt, um sie ohne Beschädigung derart gut festzuhalten, dass sie beim Einführen und an Ort Bringen im Munde des Patienten nicht verrutscht werden. Diese Aufgabe wird mit dem Halter nach Patentanspruch 1 gelöst. Weitere Ausbildungen sind in den abhängigen Ansprüchen beschrieben.

Die Erfindung wird im Einzelnen anhand von Zeichnungen eines Ausführungsbeispiels näher erläutert.
- Figur 1: zeigt den erfindungsgemässen Filmhalter in perspektivischer Sicht und
- Figur 2: zeigt einen Schnitt gemäss II/II in Fig. 1.

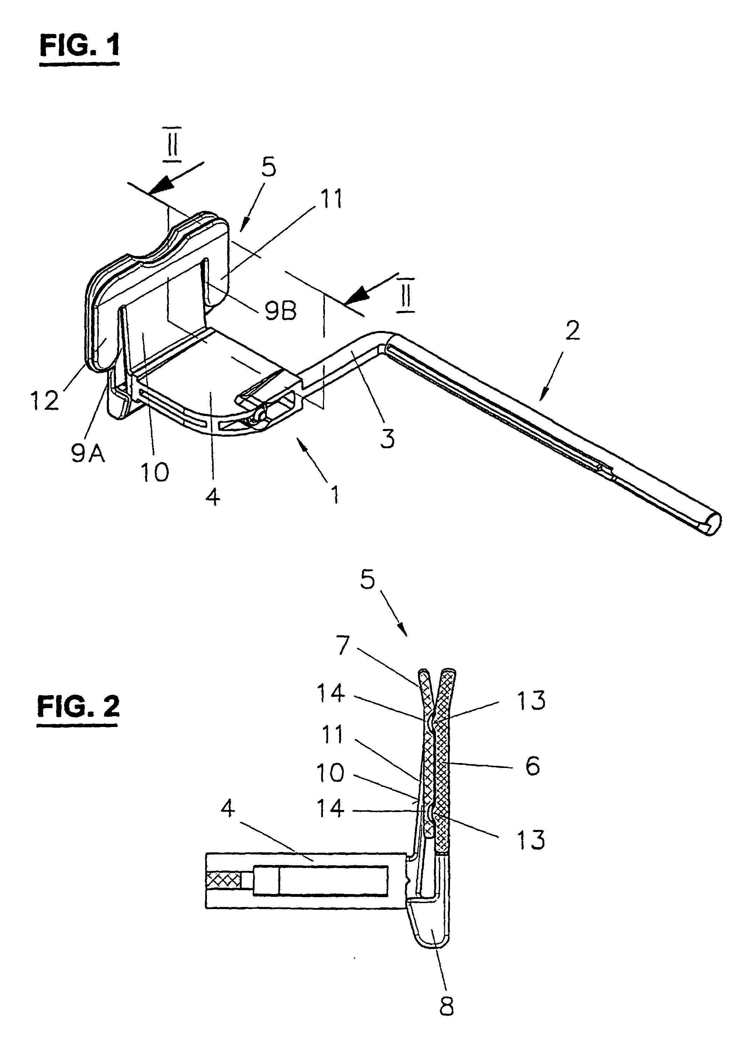

In Fig. 1 erkennt man die wesentlichen, an sich bekannten Teile des Filmhalters 1: den Indikatorstab 2 mit der Abkröpfung 3 und senkrecht zur Abkröpfung den Aufbissblock 4 mit der daran anschliessenden Filmklemme 5. Der Indikatorstab kann verschiedene Visiervorrichtungen aufnehmen, beispielsweise wie in der eingangs erwähnten EP-B-397 599 oder gemäss der CH-A-690 868.

Die Filmklemme 5 besteht, siehe auch Fig. 2, aus einer hinteren, oralen Filmplatte 6 und einer vorderen, facialen Filmplatte 7, die über ein Verbindungsteil 8, das durch Zusammenstecken der oralen und facialen Filmplatten zustande kommt, miteinander verbunden sind. Dabei ist die Breite des Verbindungsteils so gewählt, dass dort der Film oder die Folie nicht geklemmt wird. Die hintere Filmplatte 6 ist dicker und auch steifer als die vordere Platte 7 ausgeführt sowie in der Regel einstückig hergestellt. Die vordere Platte 7 ist in Abschnitte unterteilt, welche jeder für sich im voraus mit einer definierten Spannkraft ausgestattet werden, indem die unteren Schenkelteile der seitlichen Abschnitte 11 und 12 nicht in der genau gleichen Ebene wie der mittlere Abschnitt 10 liegen,so dass der Druck nicht einfach nur durch die Dicke des in die Filmklemme eingeschobenen Filmes entsteht und die Druckverteilung nicht nur an einem Punkt oder an einer Kante sondern verteilt auf verschiedene Abschnitte zustande kommt.

Die dünnere vordere Platte 7 ist von unten, d.h. vom Aufbissblock her, aufgeschnitten, wobei die beiden Schlitze 9A und 9B die vordere Platte 7 in einen mittleren Abschnitt 10 sowie zwei Seitenabschnitte 11 und 12 unterteilen. Dadurch entsteht eine faciale Platte 7, die in verschiedene Abschnitte unterteilt ist und die sich dadurch den verschiedenen Filmen, insbesondere den dünnen Speicherfolien derart anpassen kann, dass der Druck darauf über die ganze Fläche verteilt ist. Es müssen nicht notwendigerweise drei Abschnitte sein, die vordere Platte kann auch in zwei oder mehr als drei Abschnitte aufgeteilt sein, die für sich beweglich sind, um sich gut an die Dicke und Topographie des Filmes oder Folie anpassen zu können. Diese Abschnitte können auch anders als durch Schlitze, bzw. senkrechte Schlitze, voneinander getrennt sein.

Um eine bessere Klemmwirkung zu erzielen, weist die orale Platte 6 Noppen 13 auf, die mit entsprechenden Vertiefungen 14 an der facialen Platte zusammenarbeiten. In vorliegendem Ausführungsbeispiel sind vier Noppen 13 bzw. Ausnehmungen 14 eingezeichnet, doch können dies selbstverständlich auch, besonders bei kleineren Filmklemmen, weniger z.B. zwei oder drei sein, oder auch gegebenenfalls mehr.

Wie bereits eingangs erwähnt, kann die Klemmwirkung durch die Verwendung eines geeigneten Kunststoffes und durch die Wahl der Wandstärken der Platten und des Verbindungsteils 8 bestimmt und voreingestellt werden, derart, dass die Speicherfolien nicht beschädigt werden.

## Patentansprüche

1. Filmhalter für die Dentaltechnik, mit einem Aufbissblock (4) sowie einer etwa U-förmigen Filmklemme (5), wobei die Filmklemme (5) eine orale (6) und eine faciale (7) Platte aufweist, **dadurch gekeinnzeichnet, dass** die orale Platte (6) steifer ist als die faciale Platte (7) und die faciale Platte (7) ausgebildet ist, um für unterschiedliche Dicken und/oder Topographien des Filmes oder der Speicherfolie eine daran angepasste Druckverteilung zu erhalten indem die faciale Platte (7) in Abschnitte unterteilt ist, welche jeder für sich mit einer vor definierten spannkraft ausgestattet sind.

2. Filmhalter nach Anspruch 1, **dadurch gekennzeichnet, dass** die faciale Platte (7) in Abschnitten (10, 11, 12) unterteilt ist, wobei die unteren Schenkelteile der seitlichen Abschnitte (11, 12) nicht in der genau gleichen Ebene wie der mittlere Abschnitt (10) liegen

3. Filmhalter nach Anspruch 1 oder 2, **dadurch gekennzeichnet, dass** die Innenseiten der Filmklemmen-Platten (6, 7) Noppen (13), bzw. dazugehörige Vertiefungen (14) aufweisen.

4. Filmhalter nach einem der Ansprüche 1 bis 3, **dadurch gekennzeichnet, dass** er einen Indikatorstab (2) mit einer Abkröpfung (3) aufweist.

## Claims

1. Film holder for dental technique, comprising a bite portion (4) and an approximately U-shaped film clip (5), the film clip (5) comprising an oral (6) and a facial (7) plate, **characterised in that** the oral plate (6) is more rigid than the facial plate (7) and the facial plate (7) is designed such as to obtain a pressure distribution adapted to different thicknesses and/or topographies of films or digital picture plates due to the fact that the facial plate (7) is divided into sections each of which is individually provided with a predetermined resilience.

2. Film holder according to claim 1, **characterised in that** the facial plate (7) is divided into sections (10, 11, 12) where the lower shank portions of the lateral sections (11, 12) do not lie in exactly the same plane as the middle section (10).

3. Film holder according to claim 1 or 2, **characterised in that** the inner sides of the film clip plates (6, 7) are provided with knobs (13) and corresponding recesses (14), respectively.

4. Film holder according to one of claims 1 to 3, **characterised in that** it comprises an indicator rod (2) with a cranked portion (3).

## Revendications

1. Porte-films pour la technique dentaire, comportant une partie à mordre (4) et une pince porte-films (5) en forme d'U approximativement, la pince porte-films (5) comportant une plaque orale (6) et faciale (7), **caractérisé en ce que** la plaque orale (6) est plus rigide que la plaque faciale (7) et que la plaque faciale (7) présente une configuration qui permet d'obtenir une distribution de la pression adaptée à différentes épaisseurs et/ou topographies du film ou du film d'imagerie indirecte grâce au fait que la plaque faciale (7) est divisée en sections lesquelles sont dotées individuellement d'une élasticité prédéfinie.

2. Porte-films selon la revendication 1, **caractérisé en ce que** la plaque faciale (7) est divisée en sections (10, 11, 12) où la partie inférieure des pattes des sections latérales (11, 12) n'est pas exactement dans le même plan que la section centrale (10).

3. Porte-films selon la revendication 1 ou 2, **caractérisé en ce que** les côtés intérieurs des plaques (6, 7) de la pince porte-films présentent des boutons (13) resp. des évidements (14) y associés.

4. Porte-films selon l'une des revendications 1 à 3, **caractérisé en ce qu'**il présente une barre d'indication (2) avec une partie coudée (3).
